# EUROPÄISCHE PATENTSCHRIFT

(11) **EP 0 761 629 B1**
(45) Veröffentlichungstag und Bekanntmachung des Hinweises auf die Patenterteilung: **01.08.2001**
(21) Anmeldenummer: 96113619.9
(22) Anmeldetag: 26.08.1996
(51) Int. Cl.: C07C 33/02, C07C 33/025, C07C 69/007, C07C 69/07, C07C 69/145, C07C 69/24, A61K 7/46

(54) **Offenkettige olefinisch ungesättigte Verbindungen und ihre Verwendung als Riechstoffe**
Aliphatic unsaturated compounds and their use as fragrances
Composés aliphatiques insaturés ainsi que leur utilisation comme parfum

(30) Priorität: 06.09.1995 DE 19532886
(43) Veröffentlichungstag der Anmeldung: 12.03.1997
(73) Patentinhaber: HAARMANN & REIMER GMBH, D-37601 Holzminden (DE)
(72) Erfinder: Dilk, Erich, Dr., 37603 Holzminden (DE); Wörner, Peter, 37603 Holzminden (DE)
(74) Vertreter: Petrovicki, Wolfgang, Dr.

(56) Entgegenhaltungen:
- US-A- 3 296 080
- CHEMICAL ABSTRACTS, vol. 98, no. 10, 7.März 1983 Columbus, Ohio, US; abstract no. 77949, XP002018941 & SU-A-960 157 (INST. OF CHEMISTRY)
- STN INFORMATION SERVICE, FILE: REGISTRY XP002018940
- CHEMICAL ABSTRACTS, vol. 90, no. 1, 1.Januar 1979 Columbus, Ohio, US; abstract no. 5496, XP002018942 & HELV. CHEM. ACTA, Bd. 61, Nr. 5, 1978, Seiten 1856-18902, DILLENBERGER ET AL.: "Studies on the migratory aptitude of allyl groups in aliphatic carbenium ions"
- CHEMICAL ABSTRACTS, vol. 121, no. 17, 24.Oktober 1994 Columbus, Ohio, US; abstract no. 205745, XP002018943 & JP-A-06 092 884 (KURARAY)
- J. CHROMATOGR., Bd. 365, 1986, Seite 429-434 XP000608593 RUDOLFI ET AL.: "Gas Chromatographic Behaviour of Fragrances... "

## Beschreibung

Die Erfindung betrifft offenkettige verzweigte olefinisch ungesättigte Ester, zwei Verfahren zu ihrer Herstellung und ihre Verwendung als Riechstoffe.

Wegen der im allgemeinen unzureichenden Verfügbarkeit vieler natürlicher Riechstoffkomponenten, der notwendigen Anpassung an wechselnde modische Geschmacksrichtungen sowie dem ständig steigenden Bedarf an neuen Riechstoffen, die allein oder in Form von Kompositionen wertvolle Parfums bzw. Duftstoffe mit interessanten Duftnoten darstellen, besteht auch weiterhin ein Bedürfnis nach neuen Verbindungen mit wertvollen Riechstoffqualitäten.

Die US 3 296 080 beschreibt Linanol-Homologe, die als Riechstoffe in der Parfüm-, Kosmetik- oder Geschmackstoffindustrie eingesetzt werden können. 3,4,7-Tetramethyl-1,6-octadien-3-ol wird als Einzelverbindung genannt.

CA 98:77949 und SU-A-96015 beschreiben 2,3,6-Trimethyl-5-hepten-2-ol und dessen Derivate, die als Komponenten für Riechstoffe verwendet werden können.

CA 90:5496; RN 84607-59-0 beschreibt 2,3,6-Trimethyl-5-hepten-2-ol; ein Hinweis auf Riechstoffeigenschaften liegt vor.

CA 121:205745 und JP-A-06092884 beschreiben verschiedene Terpene und Terpenoide mit Riechstoffeigenschaften und ihre Herstellung.

J. Chromatogr. 365 (1986) Seiten 429 bis 434 untersucht das gaschromatographische Verhalten von Riechstoffen an Apiezon L und 1,2,3-Tris(β-cyanoethoxy) propane. Der Aktivitätskoeffizient von 2,2,5-Trimethylhexen-4-ol wird angegeben.

Überraschenderweise wurde gefunden, dass bestimmte offenkettige verzweigte olefinisch ungesättigte Ester - vorzugsweise solche vom Typ der Trimethylhexen-, Trimethylhepten- und Tetramethyloctenverbindungen - hervorragende Riechstoffeigenschaften besitzen.

Gegenstand der Erfindung sind also Verbindungen der Formel 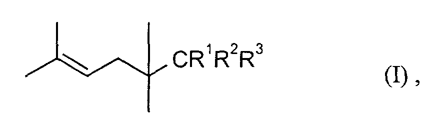 worin
- R¹: 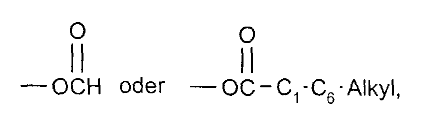
- R²: Wasserstoff oder C₁-C₄-Alkyl, und
- R³: Wasserstoff, Methyl oder Isopropyl bedeuten.

Die Alkohole (Ia) als Zwischenprodukte können durch Umsetzung der entsprechenden Carbonylverbindungen, vorzugsweise also der Aldehyde oder Ketone, mit metallorganischen Alkylierungsmitteln, wie z.B. Butyllithium oder Grignard-Verbindungen R²MgX oder R³ MgX (X = Halogen aus der Reihe Chlor, Brom, Iod) hergestellt werden:

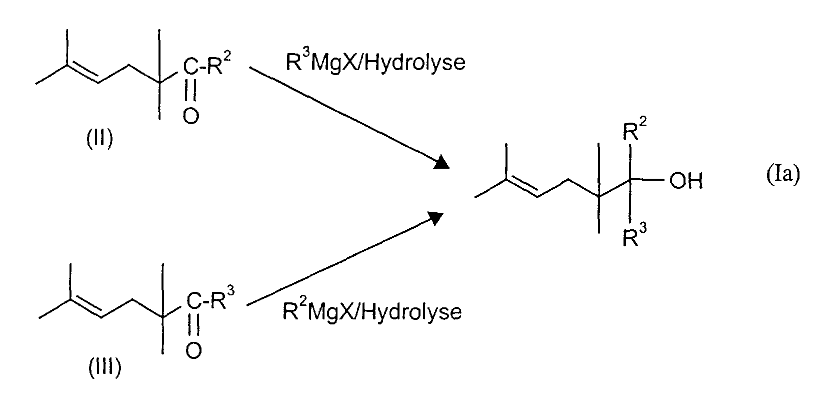

Die metallorganischen Alkylierungsmittel werden im allgemeinen in mindestens äquivalenten Mengen, bezogen auf die Carbonylverbindung (II) bzw. (III), eingesetzt.

Die Umsetzungen werden in der Regel in einem organischen Lösungsmittel, das unter Reaktionsbedingungen inert ist, durchgeführt. Geeignete inerte Lösungsmittel umfassen vor allem Ether, wie z.B. Diethylether, Dibutylether, Ethylenglykoldimethyl- und -dibutylether, Anisol, Tetrahydrofuran. Geeignet sind aber auch beispielsweise Kohlenwasserstoffe wie Hexan.

Die Umsetzung wird im allgemeinen bei Temperaturen von 20 bis 80, vorzugsweise 20 bis 65°C durchgeführt. Nach Beendigung der Reaktion und Hydrolyse kann man den entstandenen Alkohol mittels eines mit Wasser nicht mischbaren organischen Lösungsmittels aus der wässrigen Phase extrahieren.

Die Alkohole (Ia) können durch Umsetzung der Carbonylverbindungen (II) oder (III) mit einer metallorganischen Verbindung R²-Y bzw. R³-Y, wobei Y für Li oder MgX und X für Halogen aus der Reihe Chlor, Brom, Iod stehen, hergestellt werden.

Die Alkohole (Ia) können auch durch Reduktion der entsprechenden Carbonylverbindungen, vorzugsweise also der Aldehyde oder Ketone, aber auch der Ester, Säurechloride oder Carbonsäuren, mit komplexen Hydriden, wie z.B. Natrium-borhydrid, Lithiumaluminiumhydrid oder Lithiumborhydrid hergestellt werden.

Die Reduktion wird man im allgemeinen so durchführen, dass man mindestens die zum vollständigen Umsatz notwendige Menge komplexes Hydrid einsetzt, im Falle der Reduktion von Aldehyden und Ketonen also 0,25 mol, für Ester und Säurechloride 0,5 mol und für Carbonsäuren 0,75 mol komplexes Hydrid. Die Reduktion kann also folgendermaßen ablaufen:

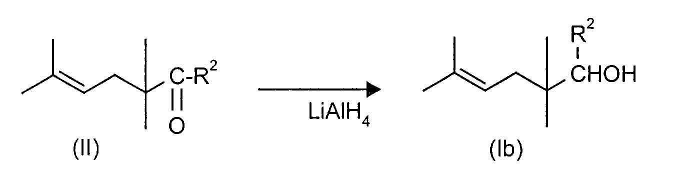

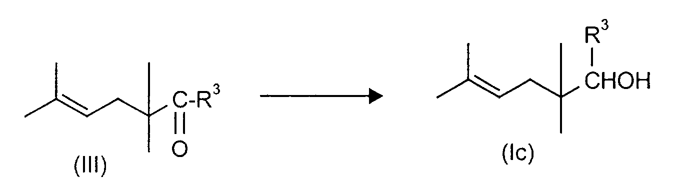

Die Reduktion kann in inerten organischen Lösungsmitteln durchgeführt werden, wie sie oben für die Reaktionen mit metallorganischen Reagentien genannt sind. Die Reaktionen können bei Temperaturen von 20 bis 90, vorzugsweise 20 bis 65°C durchgeführt werden. Nach der Hydrolyse kann die Aufarbeitung wie üblich erfolgen.

Die als Ausgangsverbindungen für die beiden beschriebenen Verfahren zu verwendenden Carbonylverbindungen sind bekannt (beispielsweise aus der US-PS 3 668 255 oder H. Mayretal., Chemische Berichte 124 (1991), Seite 203-206, VCH Verlagsgesellschaft mbH, Weinheim) oder können analog bekannten Verfahren des Standes der Technik hergestellt werden.

Aus den Alkoholen (Ia) können die entsprechenden Ester hergestellt werden. Man kann sich dabei üblicher Veresterungsmethoden bedienen, wie sie z.B. in "Organikum", Deutscher Verlag der Wissenschaften, 18. Auflage, Berlin 1990, Seite 402 f und "Methoden der Organischen Chemie" (Houben-Weyl), Band E 5, Seite 659 f, Georg Thieme Verlag, Stuttgart 1985 beschrieben sind. Ein bevorzugtes Verfahren ist die Umsetzung der Alkohole mit Carbonsäuren oder Carbonsäureanhydriden, gegebenenfalls in Gegenwart eines Veresterungskatalysators.

Bevorzugte Carbonsäuren und Carbonsäureanhydride sind z.B. Ameisensäure, Essigsäure, Propionsäure, Acetanhydrid, Propionsäureanhydrid, Buttersäureanhydrid.

Bevorzugte Veresterungskatalysatoren umfassen beispielsweise Phosphorsäure, Schwefelsäure, wasserfreien Chlorwasserstoff, Sulfonsäuren, Ionenaustauscher oder Natriumacetat

Die erfindungsgemäßen Ester der Formel (I) können also durch Veresterung der entsprechenden Alkohole der Formel (Ia) mit Carbonsäuren oder Carbonsäureanhydriden hergestellt werden.

Die erfindungsgemäßen Verbindungen der Formel (I) besitzen äußerst reizvolle Duftnoten, die sich besonders durch Frische auszeichnen und an Bergamott, Grapefruit, Agrumen erinnern. Sie lassen sich gut mit anderen Riechstoffen zu interessanten Kompositionen kombinieren, wobei die Menge vorzugsweise 1 bis 50 Gew.-%, bezogen auf die ganze Komposition, beträgt.

Außer in der Feinparfumerie können derartige Kompositionen zur Parfümierung von Kosmetika wie Cremes, Lotionen, Aerosolen, Toilettenseifen, technischen Artikeln wie Reinigungs- und Waschmitteln, Weichspülern, Desinfektionsmitteln und Textilbehandlungsmitteln dienen.

Weiterer Gegenstand der Erfindung ist also die Verwendung der Verbindungen der Formel (I) als Riechstoffe.

Die Prozentangaben in den folgenden Beispielen beziehen sich jeweils auf das Gewicht.

### Beispiele

### Beispiel 1

### 2,4,4,7-Tetramethyl-oct-6-en-3-olformiat

52 g 2,4,4,7-Tetramethyl-oct-6-en-3-ol und 65 g Ameisensäure werden 8 Stunden bei 40°C gerührt. Anschließend wird die Ameisensäure abdestilliert und das Produkt durch Spaltrohrdestillation gereinigt.

Man erhält 22,7 g (38 % d. Th.) Produkt Kp_{10mbar} = 118°C.
Geruch: Bergamott, Grapefruit, Muskatellersalbei, Agrumen

### Beispiel 2

### 2,4,4,7-Tetramethyl-oct-6-en-3-olacetat

57,6 g 2,4,4,7-Tetramethyl-oct-6-en-3-ol und 33,6 g Acetanhydrid werden unter Zusatz von 10 g Natriumacetat 5 Stunden unter Rückfluss erhitzt. Man versetzt mit Wasser, extrahiert mit tert-Butyl-methylether und destilliert über eine 30 cm Füllkörperkolonne.

Es werden 52 g (77 % d. Th.) Produkt Kp_{10mbar} = 92°C erhalten.
Geruch: Frisch, Kampfer, Bergamott, Grapefruit

### Beispiel 3

### 2,2,5-Trimethyl-hex-4-en-1-olformiat

92 g 2,2,5-Trimethyl-hex-4-en-1-ol und 130 g Ameisensäure werden 6 Stunden bei Raumtemperatur gerührt. Man versetzt mit 400 ml Wasser, trennt die Phasen und destilliert anschließend das Produkt.

Man erhält 87 g (80 % d. Th.) Produkt Kp_{10mbar} = 49°C.
Geruch: Grapefruit, Frisch, Bergamott, Styrolylacetat, Carbinol

### Beispiel 4

### 2,2,5-Trimethyl-hex-4-en-1-olpropionat

49,7 g 2,2,5-Trimethyl-hex-4-en-1-ol, 70 ml Propionsäureanhydrid und 0,1 ml Phosphorsäure werden bei Raumtemperatur gerührt. Man fügt 300 ml tert.-Butylmethylether hinzu, wäscht neutral und destilliert die organische Phase.

Es werden 66 g (95 % d. Th.) Produkt Kp_{10mbar} = 70°C erhalten.
Geruch: Frisch, grün, Bergamott, wässrig

### Beispiel 5

### 3,3,6-Trimethyl-hept-5-en-2-olformiat

Aus 46,8 g 3,3,6-Trimethyl-hept-5-en-2-ol und 60 g Ameisensäure analog Beispiel 6.

Man erhält 34,6 g (63 % d. Th.) Produkt Kp_{10mbar} = 78°C.
Geruch: Frisch, Styrolylacetat, Grapefruit, Blüte

### Beispiel 6

### 3,3,6-Trimethyl-hept-5-en-2-olpropionat

Aus 46,8 g 3,3,6-Trimethyl-hept-5-en-2-ol, 70 ml Propionsäureanhydrid und 0,1 ml Phosphorsäure analog Beispiel 7.

Es werden 52,6 g (82,3 % d. Th.) Produkt Kp_{10mbar} = 93°C erhalten.
Geruch: Frisch, Bergamott, Grapefruit

### Anwendungsbeispiel

| | A Gew.-Teile | B Gew.-Teile |
|---|---|---|
| Bergamott Identoil farblos® (H+R) | 175 | 175 |
| Dihydromyrcenol | 200 | 200 |
| Citronenöl | 100 | 100 |
| Vertocitral® (H+R) | 5 | 5 |
| Methyldihydrojasmonat | 50 | 50 |
| Citrophoral Supra® (H+R) | 5 | 5 |
| Lavandinöl Grosso | 25 | 25 |
| Muskateller Salbeiöl Franz. | 10 | 10 |
| Geranium Chin. Synthessence® (H+R) | 30 | 30 |
| Damascon Beta® (Firmenich) | 1 | 1 |
| Precyclemone B® (IFF) | 19 | 19 |
| Isoananat® (H+R) | 15 | 15 |
| Sandolen H&R® (H+R) | 5 | 5 |
| Cedrylketon® (H+R) | 100 | 100 |
| Chromanolid 50 % in DEP® (H+R) | 100 | 100 |
| Evemyl® (Givauden Roure) | 10 | 10 |
| Ambroxid rein® (H+R) | 10 | 10 |
| Dipropylenglykol | 140 | 100 |
| 2,2,5-Trimethyl-hex-4-en-1-olformiat | - | 40 |
| DEP = Diethylphthalat | | |

Durch die Zugabe von 2,2,5-Trimethyl-hex-4-en-1-olformiat wird eine unverkennbare frische grüne Spitze in der herben Komposition bewirkt.

## Patentansprüche

1. Verbindungen der Formel 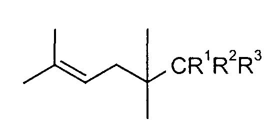 worin
R¹ 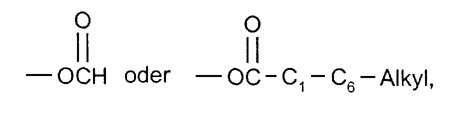 bedeutet,
R² Wasserstoff oder C₁-C₆-Alkyl und
R³ Wasserstoff, C₁-C₄-Alkyl bedeutet.

2. Verbindungen nach Anspruch 1, worin
R¹ 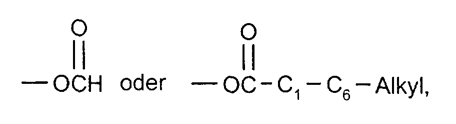 bedeutet,
R² Wasserstoff oder C₁-C₄-Alkyl und
R³ Wasserstoff, Methyl oder Isopropyl bedeutet.

3. Riechstoffzusammensetzungen, enthaltend eine Verbindung nach Anspruch 1.

## Claims

1. Compounds of the formula 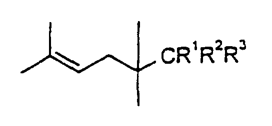 wherein
R¹ represents 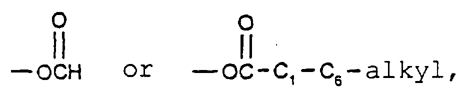
R² represents hydrogen or C₁-C₆-alkyl, and
R³ represents hydrogen, C₁-C₄-alkyl.

2. Compounds according to claim 1, wherein
R¹ represents 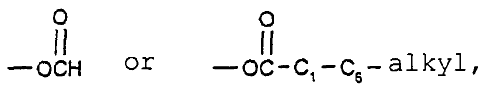
R² represents hydrogen or C₁-C₄-alkyl, and
R³ represents hydrogen, methyl or isopropyl.

3. Aromatic substance compositions containing a compound according to claim 1.

## Revendications

1. Composés de formule 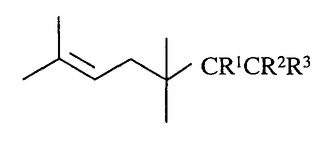 dans laquelle
R¹ représente 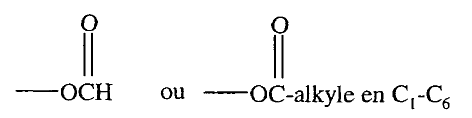
R² représente l'hydrogène ou un groupe alkyle en C₁-C₆, et
R³ représente l'hydrogène, un groupe alkyle en C₁-C₄.

2. Composés selon la revendication 1, pour lesquels
R¹ représente 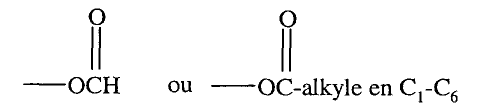
R² représente l'hydrogène ou un groupe alkyle en C₁-C₄, et
R³ représente l'hydrogène, un groupe méthyle ou isopropyle.

3. Compositions de parfums contenant un composé selon la revendication 1.
